(19) Europäisches Patentamt
European Patent Office
Office européen des brevets

(11) Publication number : **0 495 716 A2**

(12) # EUROPEAN PATENT APPLICATION

(21) Application number : **92400109.2**

(22) Date of filing : **15.01.92**

(51) Int. Cl.$^5$ : **A61B 5/05,** A61B 5/0476

(30) Priority : **15.01.91 CU 491**

(43) Date of publication of application : **22.07.92 Bulletin 92/30**

(84) Designated Contracting States : **AT BE CH DE DK ES FR GB GR IT LI LU NL SE**

(71) Applicant : **CENTRO DE NEUROCIENCIAS DE CUBA**
**Calle 25 No. 15202, esq. 158, Cubanacan, Playa**
**La Habana 12100 (CU)**

(72) Inventor : **Gonzalez Andino, Sara**
**Ave 224, Edificio no2**
**La Coronela, La Lisa (CU)**
Inventor : **Pascual Marqui, Roberto**
**Calle 26 no29**
**Vedado, La Habana (CU)**
Inventor : **Grave de Peralta Menendez, Rolando**
**Ave 224, Edificio no2**
**La Coronela, La Lisa (CU)**
Inventor : **Juan C. Jimenez, Sobrino**
**Calle 32 no62**
**Cojimar, La Habana (CU)**

(74) Representative : **Joly, Jean-Jacques et al**
**CABINET BEAU DE LOMENIE 55, rue d'Amsterdam**
**F-75008 Paris (FR)**

(54) **Method and system for the anatomic deconvolution of brain and heart electromagnetic activity.**

(57) A method and system is described for the anatomic deconvolution of brain and heart electromagnetic activity including a set of bioelectromagnetic amplifiers, NMR or TAC images from the subject's head or torso, and a computer based system for the recording and processing of human electromagnetic-physiological signals. Signals recorded on the surface of the body (head or torso) are transformed, using the information provided by the anatomic images, to an infinite homogeneous volume conductor. Generator localization and display of magnitudes of interest are performed in this medium, which is the simplest from the physical point of view.

EP 0 495 716 A2

The present invention relates to a method and system for the anatomic deconvolution of brain and heart electromagnetic activity defined here as the determination of brain/heart activity generators, and the elimination of the geometrical and conductivity effects (volume conduction effects) associated with the specific volume conductor considered, which can be the head in the case of the brain or the torso in the case of the heart.

The determination of the location and temporal evolution of the current generators of electroencephalogram (EEG), magnetoencephalogram (MEG), electrocardiogram (EKG), and magnetocardiogram (MKG), is extremely important in the diagnosis of diseases of the Central Nervous System or Heart and in brain or heart function research. Fundamental understanding of the structural organization of primary areas in the human brain can be achieved by using the previous techniques arid they have proved to be a powerful diagnostic tool for brain and heart diseases. Typical examples are the surgical treatment of epilepsy and some psychophysiological studies in the case of the brain and the noninvasive detection of the sites of the myocardial infarction in the case of the heart.

A very important aspect when localizing these generators is the selection of a proper model for the volume conductor. High accuracy in source localization can only be achieved when the volume conductor description is also accurate. However, very simple geometrical representations of the head and the torso had been commonly used, due to the mathematical simplicity that they introduce. One example is the widely used one layer spherical model which is only a first approximation to the realistic head or torso shape and therefore the accuracy in source localization may be significantly reduced. Some previous attempts of improving brain activity generator localization considering the existence of several layers of different conductivity in the spherical model have been made (J.P. Ary, S.A. Klein, DH Fender, IEEE Transactions on Biomedical Engineering, 1981,BME-28:447-452; M. Scherg, D von Cramon, Electroencephalography arid Clinical Neurophysiology, 1985, 62:32:44). The main limitation of this kind of models is that the true volume conductor geometry is neglected. First attempts of anatomical deconvolution has been previously made by transforming the measured potentials into potentials measured on the inner conductivity interfaces (Yamashita Y, IEEE Transaction Biomedical Engineering, 1982, 29:791:724). Mostly, these kind of methods are not efficient enough and do not improve considerably generator localization to be applied in clinical daily routine.

A realistic model for the head or the torso (e.g. obtained from NMR images) should be used for more accurate source localization. However, the method for source localization requires in such a case much more computation (e.g., Boundary Element Method BEM, Barnard, ACL, Duck IM arid Lynn MS. Biophysical Journal, 1967:7:443462). This might be a serious drawback when exploring many different source models in a wide scale clinical screening.

Volume conductor effects also affect quantitative electromagnetic signal analysis (e.g., neurometrics) arid topographic mapping. The influence of differences in the anatomical arid conductivity properties existing in the human population may obscure the interpretation of the results, introducing effects not related to the actual physiological generators. For example, statistically independent generators produce highly correlated electromagnetic signals due to the volume conductor, producing a "blurring" effect which decreases localization accuracy. This problem can be solved by the anatomic deconvolution described here.

Combination of information provided by magnetic and electric measurements can considerably improve generator localization. However, in volume conductors of arbitrary shape, magnetic fields are dependent on the electrical potential values in the conductivity interfaces. Deconvolution of electric and magnetic fields to the infinite volume conductor introduces a complete independence in the information about the generators provided by both fields. This means, that in an unbounded homogeneous medium, only the vortex sources (component of the primary source with zero divergence) contribute to the magnetic field, and only the flow sources (component of the primary source with zero curl) contribute to the electrical potential (Williamson SJ and Kaufman L. J of Magnetism and Magnetic Materials 1981:22:129-201.).

The invention is related to a method and system for eliminating volume conduction effects from the electromagnetic activity recorded from the head (torso) surface in a subject. The system includes an array of at least 19 sensors for recording EEG, MEG, EKG, or MKG traces from the subject. The procedure can be used for simplifying the calculation of inverse solution using a method in which the volume conductor model and the source model are treated separately. First, realistic geometry and conductivity effects are eliminated by transforming the actual measured data into equivalent infinite homogeneous conductor measurements. This is performed by calculating the matrix which represents the linear transformation between the actual measured data and the infinite volume conductor. Secondly, the new equivalent data can be used for inverse solution computations in the simplest of all physical models. The method also includes:

a) an efficient procedure for calculating the transformation matrices,

b) quantitative electromagnetic signal analysis based on data free from volume conductor effects, and

c) visual display of topographic maps based on electromagnetic data also free from volume conductor effects.

The methods described here provide a means for analyzing data independent of the anatomical and electrical variability existing among humans. Also, the proposed methods reduces the inverse problem to the physical situation in which magnetic fields and voltages yield information about the components of the generators that are complementary and electromagnetically independent.

According to the first aspect of the invention, there is provided a method for the determination of brain/heart activity generators, and for the elimination of the geometrical arid conductivity effects (volume conduction effects) associated with the specific volume conductor considered, said method including the steps of:

a) Attaching or approximating a set of electrodes and magnetic sensors to the scalp or torso of a patient to detect electromagnetic physiological activity in the form of electroencephalogram (EEG), magnetoencephalogram (MEG), electrocardiogram (EKG), and magnetocardiogram (MKG);

b) Amplifying the said electromagnetic signals detected at each electrode arid magnetic sensor;

c) Obtaining on-line digital spatio-temporal signals, consisting of said EEG, MEG, EKG, arid MKG, by connecting analog-digital converters to each amplifier, and digitizing all data as it is gathered;

d) Obtaining sets of said spatio-temporal signals for the patient.

e) Describing the head (torso) shape based on the processing of the individual or standard NMR or TAC images.

f) Transforming the recorded signals fields in equivalent measurements in an infinite homogeneous volume conductor.

g) Localizing the generators of the measured activity in the infinite medium for a given source model.

h) The quantitative electromagnetic signal analysis based on data free from volume conductor effects, and the visual display of the corresponding topographic maps (e.g., neurometric measures and associated derivatives such as the surface Laplacian or Current Source Density).

In accordance with a second aspect of the invention there is provided a system for the determination of brain/heart activity generators, and for the elimination of the geometrical and conductivity effects (volume conduction effects) associated with the specific volume conductor considered, said system including:

a) A set of electrodes and/or magnetic sensors adapted to be attached or approximated to the scalp or torso of a patient in order to detect electromagnetic physiological activity in the form of electroencephalogram (EEG), magnetoencephalogram (MEG), brain evoked response (ER), electrocardiogram (EKG), and magnetocardiogram (MKG);

b) A system for the amplification of the aforementioned physiological activity;

c) Means for the analog-to-digital conversion of the preamplified physiological activity in the form of digital spatio-temporal signals, representative of the aforementioned EEG, MEG, EKG, and MKG;

d) Means for storing sets of said spatio-temporal signals for the patient and sets of NMR or TAC images, and for computing the description the head (torso) shape based on the processing of the NMR or TAC images.

e) Means for computing the transformation of the recorded signals fields in equivalent measurements in an infinite homogeneous volume conductor.

f) Means for Localizing the generators of the measured activity in the infinite medium for a given source model.

g) Means for computing the quantitative signal analysis based on data free from volume conductor effects, and for the visual display of the corresponding topographic maps (e.g., neurometric measures and associated derivatives such as the surface Laplacian or Current Source Density).

In order that the invention may be better understood, further detailed description follows with reference to the accompanying drawings in which:

Figure 1 is a block schematic drawing of one embodiment of the present invention.

In what follows an isotropic volume conductor of arbitrary geometry composed of several homogeneous layers of different conductivity will be considered. In this case, the infinite medium fields can be expressed as functions of the measured fields and of the electric potential in the conductivity interfaces, as derived by Geselowitz (Geselowitz DB. IEEE Trans Magn 1970, 2:346-347) arid Barnard et al. (Barnard ACL, Duck IM and Lynn MS. Biophys J 1967:7:443-462).

Formally, the information required to solve the surface integrals can be provided by calculating the electric potentials on each conductivity interface, and by interpolating the finite set of measurements over the scalp surface. As has been established in electrocardiography, linear relations exist between the electric potential measurements and the potentials on the inner conductivity interfaces:

$$V_k = H_k V_m$$

where H is a matrix that depends only on the volume conductor properties and on the locations of the measurements. Using these relations, the surface integrals can be solved numerically, giving the following discrete equations :

$$B_\infty = B_m + C\,V_m$$
$$V_\infty = D\,V_m$$

where the matrices C and D also depend only on the volume conductor properties and on the locations of the measurements.

The important fact to note here is that these relations are linear. The rather heavy computational burden for obtaining the transformation matrices can be completely avoided using several methods. One of them is: First consider the electric potential case only. For a given set of known current densities (e.g., N dipole sources with different locations and moments), the direct problem (Barnard's equation) can be solved numerically (e.g, using Boundary Element Method, Projective Methods) to obtain $[V_m]_i$, (i=1,2,..,N). Also, $[V_\infty]_i$ can be computed very simply in the infinite homogeneous volume conductor. If the number of sources is larger than the number of measurement locations, then the least squares estimator for the matrix D is :

$$D = \left(\sum_i [V_\infty]_i\ [V_m]_i\right)\left(\sum_i [V_m]_i\,[V_m]_i^T\right)^{-1}$$

where $X^T$ denotes the transpose of X.

Now consider the caseofsimultaneouselectricandmagnetic measurements. The same kind of procedure is valid, except that the direct magnetic problem must now be solved numerically (Geselowitz's equation). This gives:

$$C = \left(\sum_i \left([B_\infty]_i - [B_m]_i\right)\ [V_m]_i\right)\left(\sum_i [V_m]_i\,[V_m]_i^T\right)^{-1}$$

It should be emphasized that when $N \rightarrow \infty$, the proposed method for estimating matrices C and D is completely independent of the selected sources.

In the case of a given realistic head, the transformation matrices need to be calculated only once, and the inverse problem can be solved for a given primary source model (J) by using the following equations, independent of the volume conductor:

$$B_\infty(r) = \frac{\mu 0}{4\pi}\int J(r') \times \frac{(r-r')}{|r-r'|^3}\,d^3r'$$
$$V_\infty(r) = \frac{1}{4\pi\sigma}\int J(r')\,\frac{(r-r')}{|r-r'|^3}\,d^3r'$$

Previous equations, correspond to the simplest possible physical model for the volume conductor. In this situation, a variety of source models arid constraints necessary for the uniqueness of solution can easily be tested.

Since the transformation matrices are time invariant, they do not need to be recalculated when using time varying source models. For this case the procedure would be first, to transform the time series data into equivalent infinite volume conductor data, and second, to fit the time varying source model using the simple equations associated to this kind of volume conductor.

For several typical head geometries (ranging through normal head shapes among humans) arid a fixed standard set of measurement locations, the transformation matrices can be calculated and published for routine use. If measurements are made on other sites different from the fixed set, then an appropriate interpolation function can be used and evaluated at the standard locations.

The main advantages provided by the method are the following:

1) The linear transformation matrices convert the measured electric potential and magnetic field values in the actual realistic volume conductor into "equivalent infinite homogeneous volume conductor measurements". The main advantage implied by this fact is that in an unbounded homogeneous medium, only the vortex sources (component of the primary source with zero divergence) contribute to the magnetic field, and only the flow sources (component of the primary source with zero curl) contribute to the electrical potential (Williamson SJ and Kaufman L. J of Magnetism and Magnetic Materials 1981:22:129-201.) Therefore the proposed method reduces the inverse problem to the physical situation in which magnetic fields and voltages yield information about the components of the primary sources that are complementary and electromagnetically independent.

2) The linear transformation matrices are calculated only once for a given volume conductor model and a given set of measurement locations.

3) Since the transformation matrices are time invariant, they do not need to be recalculated when using time varying source models.

4) This procedure offers more accurate inverse solutions than with the spherical approximation. Furthermore, from a computational point of view, it is in general much more simple to use than the spherical model. In addition, this procedure will allow to explore many different source models in realistic geometry, efficiently enough to be applied in routine clinical practice.
5) The topographic display of the electrical potential and magnetic fields, and other derived magnitudes, in the infinite medium will allow a more clear picture of the activity not distorted by the low conductivity of the bone. Quantitative analysis of the electromagnetic activity displayed in the infinite homogeneous volume conductor, will be absolutely independent of the variability in the anatomical and electrical variability existing among humans.

## Claims

1. A method for the anatomic deconvolution of brain (heart) electromagnetic fields comprising
   a) The description of the head shape based on the processing of the individual NMR or TAC images, or based on the selection of a standard head from a database according to certain external anatomical measurements of the subject's head. The description can be performed using either a parametric model or a triangulation of the volume.
   b) The transformation of the measured fields into equivalent measurements in an infinite homogeneous volume conductor by means of the calculation of the potentials generated by a previously defined set of sources, and the calculation of the matrix associated to the linear transformation existing between these two sets of measurements.
   c) The localization of the generators of the measured activity in the infinite medium for a given source model, the display of the deconvoluted fields , associated derivatives (e.g., the surface Laplacian or Current Source Density), and other quantitative measures (e.g., as in neurometrics) in infinite medium.

2. The method of claim 1, wherein the determination of the surface representation is carried out according to the following steps
   a) Using the images of the head shape provided by the scanner, the construction of anatomical databases for typical subjects ranging through normal head shapes among humans.
   b) If individual images are not available, a standard model is selected from the database according to external anatomic measurements of the subject's head, otherwise the individual images can be employed.
   c) From the selected image, a set of discrete points is chosen in order to construct the surface representation either by means of a parametric description or by using a triangulation of the volume and the surface.
   d) The surfaces can be described, when using a parametric description, as a finite expansion in some basis functions. Spherical Harmonics or 3D Fourier descriptors are appropriate for this objective.

3. The method of claim 1, wherein the obtention of the matrix for the transformation of the measured field into an equivalent infinite medium is carried out according to the following steps
   a) Selection of a set of suitable sources e.g., monopoles or dipoles inside the volume conductor model.
   b) Calculation of the infinite medium potentials and magnetic fields generated by the selected set of sources.
   c) Numerical calculation of the potentials and magnetic fields generated by the selected sources on the measurements sites using the surface description as in claim

4. The numerical calculation can be performed using the Boundary Element Method, The Finite Element Method or the Projective Method.
   d) Calculation with some minimum error norm algorithm of the linear transformation matrix which relates the infinite medium fields and the fields on the measurement sites which were calculated in the previous steps. This is done only once and the matrix is stored for subsequent use with this subject or in a database for the standard head shape.

5. The method of claim 1, wherein the transformation of the actual measurements into infinite medium fields is carried out by aplying the linear transformation, either using the individual matrix or the standard head shape matrix which can be calculated according to the previous steps or selected from a database.

6. The method of claim 1, wherein the localization of the generators of the measured activity is carried out

in the infinite medium or when the quantitative analysis of the fields and/or the topographical mapping is performed for this kind of medium in which the volume conductor effects are eliminated.

**7.** A system for the determination of brain/heart activity generators, and for the elimination of the geometrical and conductivity effects (volume conduction effects) associated with the specific volume conductor considered, wherein said system includes:

a) A set of electrodes and/or magnetic sensors adapted to be attached or approximated to the scalp or torso of a patient in order to detect electromagnetic physiological activity in the form of electroencephalogram (EEG), magnetoencephalogram (MEG), brain evoked response (ER), electrocardiogram (EKG), and magnetocardiogram (MKG);

b) A system for the amplification of the aforementioned physiological activity;

c) Means for the analog-to-digital conversion of the preamplified physiological activity in the form of digital spatio-temporal signals, representative of the aforementioned EEG, MEG, EKG, and MKG;

d) Means for storing sets of said spatio-temporal signals for the patient and sets of NMR or TAC images, and for computing the description the head (torso) shape based on the processing of the NMR or TAC images.

e) Means for computing the transformation of the recorded signals fields in equivalent measurements in an infinite homogeneous volume conductor.

f) Means for localizing the generators of the measured activity in the infinite medium for a given source model.

g) Means for computing the quantitative signal analysis based on data free from volume conductor effects, and for the visual display of the corresponding topographic maps (e.g., neurometric measures and associated derivatives such as the surface Laplacian or Current Source Density).

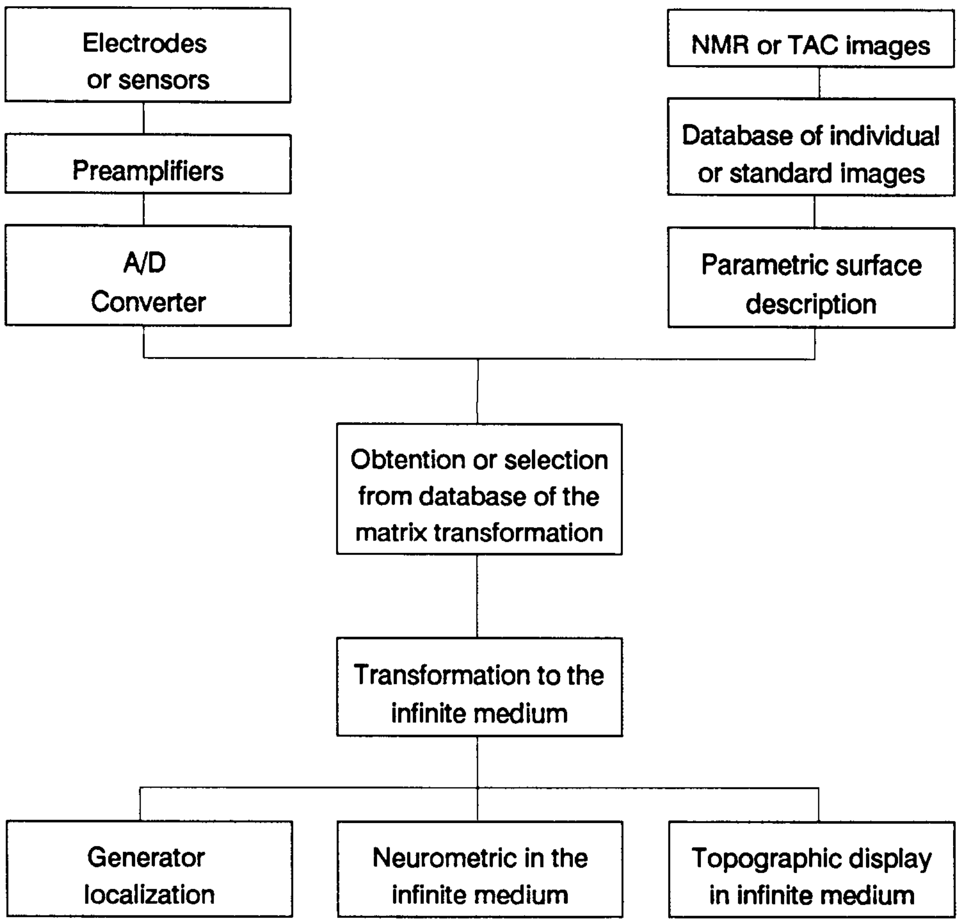
Electrodes
or sensors
Preamplifiers
A/D
Converter
NMR or TAC images
Database of individual
or standard images
Parametric surface
description
Obtention or selection
from database of the
matrix transformation
Transformation to the
infinite medium
Generator
localization
Neurometric in the
infinite medium
Topographic display
in infinite medium

Fig. 1